# DEMANDE DE BREVET EUROPEEN

(11) **EP 2 353 577 A2**
(43) Date de publication de la demande: **10.08.2011**
(21) Numéro de dépôt: 10194847.9
(22) Date de dépôt: 14.12.2010
(51) Int. Cl.: A61K 8/06, A61K 8/31, A61K 8/894, A61K 8/895

(54) **Emulsion e/h comprenant un élastomère de silicone émulsionnant et un alcane linéaire volatil**

(30) Priorité: 18.12.2009 FR 0959218
(71) Demandeur: L'Oréal, 75008 Paris (FR)
(72) Inventeur: Dumousseaux, Christophe, 92160, Antony (FR); Dique-Mouton, Valérie, 94550, Chevilly Larue (FR)
(74) Mandataire: Leonard, Armelle

(57) **Abrégé**

La présente invention concerne une composition cosmétique de soin et/ou de maquillage des matières kératiniques, en particulier la peau, sous la forme d'une émulsion eau-dans huile comprenant, dans un milieu physiologiquement acceptable:
(i) au moins un ou plusieurs alcane(s) linéaire(s) volatil(s) notamment en C7-C14, et
(ii) au moins un élastomère de silicone émulsionnant.

## Description

La présente invention a pour objet une composition cosmétique de soin et/ou maquillage sous la forme d'une émulsion eau dans huile contenant un élastomère de silicone émulsionnant et un ou plusieurs alcane(s) volatil(s) linéaire(s). Une telle composition présente à la fois une bonne stabilité, un caractère fluide, une facilité d'application et un résultat final doux et matifiant.

Les compositions de soin et/ou maquillage, en particulier les fonds de teint, se présentent sous forme de textures variées allant du fluide au solide.
Les textures fluides sont attractives pour la consommatrice car faciles à appliquer et procurant une sensation de film fin et un fini léger et naturel qui se fait oublier après l'application.

Il est néanmoins difficile d'obtenir des textures fluides sous la forme d'émulsions eau-dans-huile, avec une teneur en phase aqueuse importante, recherchée pour ses bénéfices fraîcheur, hydratation et effet non gras, qui a tendance à épaissir la composition. Par ailleurs, les textures fluides, en particulier lorsqu'elles contiennent des matières pulvérulentes, notamment des particules de densité élevée (ex : pigments, charges minérales) présentent des problèmes de stabilité au cours du temps. Et lorsque le produit est destiné à procurer de la matité et de la longue tenue et qu'il contient un fort taux de matières pulvérulentes et éventuellement la présence d'un agent filmogène, le temps de séchage est généralement très court ce qui empêche de travailler suffisamment longtemps le produit sur la peau.

Il semble donc intéressant de pouvoir développer des compositions de soin et/ou de maquillage matifiantes , alliant une bonne fluidité, une bonne stabilité, et qui soient aptes à être travaillés longtemps sur la peau en donnant un fini doux et matifiant.

La Demanderesse a trouvé de manière surprenante que l'utilisation d'un ou plusieurs alcanes linéaires volatils en C7-C14, notamment avec un point éclair >70°C, permettait de fluidifier une composition contenant un élastomère de silicone émulsionnant et ce même avec un taux de phase aqueuse très important (ratio phase aqueuse/phase grasse liquide >1). L'association selon l'invention permet donc de formuler des émulsions eau-dans-huile fluides avec un fort taux de phase aqueuse.

La Demanderesse a par ailleurs montré que l'utilisation d'un ou plusieurs alcanes linéaires volatils, notamment avec un point éclair >70°C, et d'au moins un élastomère de silicone émulsionnant, permettait d'obtenir un produit contenant des matières pulvérulentes avec une bonne fluidité, une bonne stabilité, de bonnes propriétés d'application et un effet doux et matifiant après l'application.

L'invention a donc pour objet, selon un premier aspect, une composition cosmétique de soin et/ou de maquillage des matières kératiniques, en particulier la peau, sous la forme d'une émulsion eau-dans huile comprenant, dans un milieu physiologiquement acceptable:
(i) au moins un ou plusieurs alcane(s) linéaire(s) volatil(s) notamment en C7-C14,
(ii) au moins un élastomère de silicone émulsionnant.

Selon un premier mode de réalisation, l'invention concerne une composition cosmétique de soin et/ou de maquillage des matières kératiniques, en particulier la peau, sous la forme d'une émulsion eau-dans huile comprenant, dans un milieu physiologiquement acceptable:
(i) au moins un ou plusieurs alcane(s) linéaire(s) volatil(s) notamment en C7-C14,
(ii) au moins un élastomère de silicone émulsionnant, et
(iii) au moins 10% en poids de matières pulvérulentes, par rapport au poids total de ladite composition.

Selon un autre mode de réalisation, l'invention concerne une composition cosmétique de soin et/ou de maquillage des matières kératiniques, en particulier la peau, sous la forme d'une émulsion eau-dans huile comprenant, dans un milieu physiologiquement acceptable:
(i) au moins un ou plusieurs alcane(s) linéaire(s) volatil(s) notamment en C7-C14, et
(ii) au moins un élastomère de silicone émulsionnant, et caractérisée en ce que le ratio phase aqueuse/ phase grasse liquide est supérieur à 1.

Selon un autre mode de réalisation, l'invention concerne une composition cosmétique de soin et/ou de maquillage des matières kératiniques, en particulier la peau, sous la forme d'une émulsion eau-dans huile comprenant, dans un milieu physiologiquement acceptable:
(i) au moins un ou plusieurs alcane(s) linéaire(s) volatil(s) notamment en C7-C14,
(ii) au moins un élastomère de silicone émulsionnant, et
(iii) au moins 10% en poids de matières pulvérulentes, par rapport au poids total de ladite composition, et
   caractérisée en ce que le ratio phase aqueuse/ phase grasse liquide est supérieur à 1.

La présente invention vise également un procédé cosmétique de soin et/ou de maquillage des matières kératiniques, en particulier la peau, comprenant l'application d'une composition selon l'invention.

La composition selon l'invention est sous la forme d'une émulsion eau-dans-huile, comprenant une phase aqueuse dispersée dans une phase grasse liquide, et comprend au moins un milieu physiologiquement acceptable.
Par « milieu physiologiquement acceptable », on entend désigner un milieu convenant particulièrement à l'application d'une composition de l'invention sur les matières kératiniques, notamment la peau et les lèvres.
Le milieu physiologiquement acceptable est généralement adapté à la nature du support sur lequel doit être appliquée la composition, ainsi qu'à l'aspect sous lequel la composition doit être conditionnée.

La composition selon l'invention comprend au moins un ou plusieurs alcanes linéaires volatils notamment en C7-C14.

### ALCANES LINEAIRES VOLATILS

La composition de l'invention peut comprendre de 1 à 30%, de préférence de 2 à 15%, de préférence de 3 à 10% en poids d'alcanes linéaires volatils par rapport au poids total de ladite composition.

Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm²/min, à température ambiante (25°C) et pression atmosphérique (760 mm Hg).

De façon préférée, les « alcanes linéaires volatils » convenant à l'invention présentent une vitesse d'évaporation allant de 0,01 à 3,5 mg/cm²/min, à température ambiante (25°C) et pression atmosphérique (760 mm Hg).

De façon préférée, les « alcanes linéaires volatils » convenant à l'invention présentent une vitesse d'évaporation allant de 0,01 à 1,5 mg/cm²/min, à température ambiante (25°C) et pression atmosphérique (760 mm Hg).

De façon plus préférée, les « alcanes linéaires volatils » convenant à l'invention présentent une vitesse d'évaporation allant de 0,01 à 0,8 mg/cm²/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,3 mg/cm²/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,12 mg/cm²/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les « alcanes linéaires volatils » convenant à l'invention ont une pression de vapeur (appelée également pression de vapeur saturante) non nulle, à température ambiante, en particulier une pression de vapeur allant de 0,3 Pa à 6000 Pa.

De façon préférée, les « alcanes linéaires volatils » convenant à l'invention ont une pression de vapeur allant de 0,3 à 2000 Pa, à température ambiante (25°C).

De façon préférée, les « alcanes linéaires volatils » convenant à l'invention ont une pression de vapeur allant de 0,3 à 1000 Pa, à température ambiante (25°C)

De façon plus préférée, les « alcanes linéaires volatils » convenant à l'invention ont une pression de vapeur allant de 0,4 à 600 Pa, à température ambiante (25°C).

De façon préférée, les « alcanes linéaires volatils » convenant à l'invention ont une pression de vapeur allant de 1 à 200 Pa, à température ambiante (25°C).

De façon encore préférée, les « alcanes linéaires volatils » convenant à l'invention ont une pression de vapeur allant de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, un alcane convenant à l'invention peut être un alcane linéaire volatil comprenant de 7 à 14 atomes de carbone.

De façon préférée, les « alcanes linéaires volatils » convenant à l'invention comprennent de 8 à 14 atomes de carbone.

De façon préférée, les « alcanes linéaires volatils » convenant à l'invention comprennent de 9 à 14 atomes de carbone.

De façon préférée, les « alcanes linéaires volatils » convenant à l'invention comprennent de 10 à 14 atomes de carbone.

De façon préférée, les « alcanes linéaires volatils » convenant à l'invention comprennent de 11 à 14 atomes de carbone.

Selon un mode de réalisation avantageux, les « alcanes linéaires volatils » convenant à l'invention présentent une vitesse d'évaporation, telle que définie plus haut, allant de 0,01 à 3,5 mg/cm²/min, à température ambiante (25°C) et pression atmosphérique (760 mm Hg), et comprennent de 8 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14), en particulier l'isotope ¹⁴C peut être présent en un ratio ¹⁴C /¹²C supérieur ou égal à 1.10⁻¹⁶, de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C / ¹²C va de 6.10⁻¹³ à 1,2.10⁻¹².

La quantité de d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets de la société Cognis WO 2007/068371, ou W02008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus aux exemples 1 et 2 de la demande W02008/155059 de la Société Cognis.

On peut également citer le n-dodécane (C12) et le n-tétradécane (C14) vendus par Sasol respectivement sous les références PARAFOL 12-97 et PARAFOL 14-97, ainsi que leurs mélanges.

On pourra utiliser l'alcane linéaire volatil seul.
On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un premier mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que par exemple un mélange d'au moins 3 alcanes linéaires volatils comportant de 7 à 14 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cn avec n allant de 7 à 14
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14, par rapport au poids total des alcanes dans ledit mélange.

En particulier, ledit mélange d'alcanes selon l'invention contient :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0.1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane)
- de 20 à 45% en poids, de préférence de 24 à 40% en poids d'alcane linéaire volatil en C13 (n-tridécane) par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

Selon un mode particulier de l'invention, la composition peut comprendre moins de 15% en poids, voire moins de 10% en poids, voire moins de 5% en poids, voire être dépourvue d'huile siliconée cyclique.

Le ou les alcanes linéaires volatils en C7-C14 présents dans la composition permettent d'obtenir une émulsion eau-dans-huile qui se travaille longtemps pendant l'application, malgré une teneur en matières pulvérulentes (ex : pigments, charges) supérieure à 10%, voire supérieure à 20% en poids par rapport au poids total de ladite composition.

Le ou les alcanes linéaires volatils en C7-C14 présents dans la composition permettent aussi de façon surprenante de fluidifier la composition contenant l'élastomère de silicone émulsionnant et ce même avec un taux de phase aqueuse très importante.

La composition selon l'invention comprend également au moins un élastomère de silicone émulsionnant.

### ELASTOMERES DE SILICONE

### Elastomères de silicone émulsionnants

L'élastomère de silicone émulsionnant permet d'obtenir une émulsion eau-dans-huile selon l'invention avec une bonne stabilité. Sa combinaison avec l'alcane linéaire volatil permet d'obtenir une texture à la fois très fluide et très confortable à l'application. Il procure également un toucher très doux et matifiant après l'application. Il améliore également les propriétés d'application de la composition selon l'invention.

Par élastomère de silicone émulsionnant on entend un élastomère de silicone comprenant au moins une chaîne hydrophile.

L'élastomère de silicone émulsionnant peut être choisi parmi les élastomères de silicone polyoxyalkylénés, les élastomères de silicone polyglycérolés, et leurs mélanges.

### Elastomères de silicone polyoxyalkylénés

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est de préférence mélangé avec au moins une huile hydrocarbonée et/ou une huile siliconée pour former un gel. Dans ces gels, l'élastomère polyoxyalkyléné peut être sous forme de particules non-sphériques.

Des élastomères polyoxyalkylénés sont notamment décrits dans les brevets US5236986, US5412004, US5837793, US5811487. Comme élastomère de silicone polyoxyalkyléné, on peut utiliser ceux commercialisés sous les dénominations "KSG-21", "KSG-20", "KSG-30", "KSG-31", KSG-32", "KSG-33", "KSG-210", "KSG-31 0", "KSG-320", "KSG-330", "KSG-340", "X-226146" par la société Shin Etsu, "DC9010", "DC9011" par la société Dow Corning.

Selon un mode préféré, on utilisera l'élastomère de silicone polyoxyalkyléné commercialisé sous la dénomination KSG-210 par la société Shin Etsu.

L'élastomère de silicone émulsionnant peut être également choisi parmi les élastomères de silicone polyglycérolés.

### Elastomères de silicone polyglycérolés

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁ -0-[ Gly ]n-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel. Dans ces gels, l'élastomère polyglycérolé est souvent sous forme de particules non-sphériques.

De tels élastomères sont notamment décrits dans la demande de brevet W02004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

Le ou les élastomères de silicone émulsionnants sont présents dans la composition de l'invention en une teneur allant de 0,1% à 20%, notamment de 0,2% à 10% et de préférence de 0,5% à 5% en poids par rapport au poids total de ladite composition.

Outre les élastomères émulsionnants, la composition selon l'invention peut comprendre des élastomères non émulsionnants.

### Elastomères additionnels non émulsionnants

Le terme élastomères de silicone "non émulsionnant" définit des élastomères organopolysiloxane ne contenant pas de chaîne hydrophile telle que des chaînes polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.
Les organopolysiloxanes (A2) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2). Avantageusement, la somme du nombre de groupements éthyléniques par molécule du composé (A2) et le nombre d'atomes d'hydrogène liés au silicum par molécule du composé (B2) est d'au moins 4.
Le composé (B2) peut être sous toute structure moléculaire, notamment de structure chaîne linéaire, chaîne ramifiée, structure cyclique.

Le composé (B2) peut avoir une viscosité à 25 °C allant de 1 à 50 000 centistokes, notamment pour être bien miscible avec le composé (A).
Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.
Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

L'élastomère de silicone non émulsionnant selon l'invention peut être mélangé avec au moins une huile hydrocarbonée et/ou une huile siliconée pour former un gel. Dans ces gels, l'élastomère non émulsionnant est sous forme de particules non-sphériques.

Comme élastomères non émulsionnants, on peut utiliser ceux vendus sous les dénominations "KSG-6", "KSG-15", "KSG-16", "KSG-18", "KSG-41 ", "KSG-42", "KSG-43", "KSG-44, USG-105, USG-106 par la société Shin Etsu, "DC 9040", "DC9041", "DC 9509", "DC9505", "DC 9506", "DC 5930", "DC9350", "DC9045", "DC9043" par la société Dow Corning, "GRANSIL" par la société Grant Industries, "SFE 839" par la société General Electric.
Selon un mode particulier, on utilise l'élastomère non émulsionnant commercialisé sous la dénomination KSG-16 de la société Shin Etsu.

Le ou les élastomères non émulsionnants peuvent être présents en une teneur allant de 0,1% à 20% en poids par rapport au poids total de la composition, de préférence allant de 0,2% à 10% en poids, et plus préférentiellement allant de 0,5% à 5% en poids.

Selon un mode particulier de l'invention, la composition comprend au moins un élastomère de silicone polyoxyalkyléné.

Selon un mode particulier, la composition comprend au moins un élastomère de silicone polyoxyalkyléné et au moins un élastomère de silicone non émulsionnant.

Selon un mode particulier de l'invention, la composition comprend en outre au moins 10% en poids de matières pulvérulentes, par rapport au poids total de ladite composition.

### MATIERES PULVERULENTES

Par 'matières pulvérulentes', on entend toutes particules de type matières colorantes et/ou charges, telles que définis ci-après.
En particulier, la composition comprend au moins une matière pulvérulente choisie parmi les pigments, les nacres, les charges et leurs mélanges, en particulier les pigments.
Les matières pulvérulentes sont présentes dans la composition en une teneur allant de 10 à 50% en poids, en particulier de 15 à 40% en poids et notamment de 20 à 30% en poids par rapport au poids total de ladite composition.
Lesdites matières pulvérulentes sont dispersées sous une forme homogène et stabilisée.

Selon un mode de réalisation, la composition de l'invention comprend au moins une charge.

### CHARGES

Une composition conforme à l'invention peut également comprendre au moins une charge, de nature organique ou minérale.

Par « charge », il faut comprendre les particules incolores ou blanches, solides de toutes formes, qui se présentent sous une forme insoluble et dispersée dans le milieu de la composition. De nature minérale ou organique, elles permettent de conférer à la composition de la douceur, de la matité et de l'uniformité au maquillage.

Les charges peuvent être présentes dans l'émulsion en une teneur allant de 0,5 % à 20% en poids, par rapport au poids total de l'émulsion, de préférence 2 % à 10 %.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Parmi les charges minérales utilisables dans les compositions selon l'invention, on peut citer le talc, le mica, la silice, le siloxysilicate de triméthyle, le kaolin, la bentone, le carbonate de calcium et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, et leurs mélanges.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon^{®}), la lauroyl-lysine, l'amidon, les poudres de polymères de tétrafluoroéthylène, les microsphères creuses de polymères, telles l'EXPANCEL (NOBEL INDUSTRIE), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore^{®} L 200 (Chemdal Corporation), les microbilles de résine de silicone (Tospearl^{®} de Toshiba, par exemple), les poudres de polyuréthane, en particulier, les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400^{®} ou PLASTIC POWDER D-800^{®} par la société TOSHIKI, les microcires de Carnauba, telles que celle commercialisée sous la dénomination de MicroCare 350^{®} par la société MICRO POWDERS, les microcires de cire synthétique, telles que celle commercialisée sous la dénomination de MicroEase 114S^{®} par la société MICRO POWDERS, les microcires constituées d'un mélange de cire de Carnauba et de cire de polyéthylène, telles que celles commercialisées sous les dénominations de Micro Care 300^{®} et 310^{®} par la société MICRO POWDERS, les microcires constituées d'un mélange de cire de Carnauba et de cire synthétique, telles que celle commercialisée sous la dénomination Micro Care 325^{®}par la société MICRO POWDERS, les microcires de polyéthylène, telles que celles commercialisées sous les dénominations de Micropoly 200^{®}, 220^{®}, 220L^{®} et 250S^{®} par la société MICRO POWDERS ;les fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser les fibres ont une longueur allant de 1 µm à 10 mm, de préférence de 0,1 mm à 5 mm et mieux de 0,3 mm à 3 mm. Leur section peut être comprise dans un cercle de diamètre allant de 2 nm à 500 µm, de préférence allant de 100 nm à 100 µm et mieux de 1 µm à 50 µm. A titre de fibres utilisables dans les compositions selon l'invention, on peut citer les fibres non rigides telles que les fibres de polyamide (Nylon^{®}) ou les fibres rigides telles que les fibres de polyimide-amide comme celles vendues sous les dénominations KERMEL^{®}, KERMEL TECH^{®} par la société RHODIA ou de poly-(p-phénylène-téréphtalamide) (ou d'aramide) notamment vendues sous la dénomination Kevlar^{®} par la société DUPONT DE NEMOURS,
et leurs mélanges.

Selon un mode de réalisation, la composition de l'invention comprend au moins une matière colorante pulvérulente, en particulier au moins un pigment.

### MATIERES COLORANTES PULVERULENTES

Les matières colorantes pulvérulentes peuvent être présentes à raison de 3 à 30 % en poids, notamment, de 8 à 20 % en poids, et en particulier, de 10 à 15 % en poids, par rapport au poids total de la composition cosmétique.

Les matières colorantes pulvérulentes sont notamment choisies parmi des matières colorantes pulvérulentes organiques ou inorganiques, notamment de type pigments ou nacres, des matériaux à effet optique spécifique, et leurs mélanges.

Selon un mode particulier, les matières colorantes pulvérulentes sont traitées en surface par un agent hydrophobe. L'agent de traitement hydrophobe peut être choisi parmi les silicones comme les méthicones, les diméthicones, les perfluoroalkylsilanes ; les acides gras comme l'acide stéarique; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

### Pigments

Par pigments, il faut comprendre des particules blanches ou colorées, minérales ou organiques, insolubles dans une solution aqueuse, destinées à colorer et/ou opacifier le film résultant.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

On utilise de préférence des pigments d'oxydes de fer ou de dioxyde de titane.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être, par exemple, de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Comme autres pigments, on peut également utiliser des pigments composites tels que décrits dans la demande EP 1 545 437. Ces pigments composites peuvent être composés notamment de particules comportant un noyau inorganique recouvert au moins partiellement d'un pigment organique et au moins un liant assurant la fixation des pigments organiques sur le noyau.

Le noyau inorganique peut être réalisé dans un matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silices, les silicates, notamment les aluminosilicates et les borosilicates, le mica synthétique, et leurs mélanges. Selon un mode particulier, le noyau inorganique est un oxyde de titane.

La matière colorante organique peut comporter par exemple des pigments organiques qui peuvent être choisis parmi les composés ci-dessous et leurs mélanges :
- le carmin de cochenille,
- les pigments organiques de colorants azoïques, anthraquinoniques, indigoïdes, xanthéniques, pyréniques, quinoliniques, de triphénylméthane, de fluorane, les laques organiques ou sels organiques insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Selon un mode particulier, on utilise le pigment organique D&C Red n° 7.

Selon un autre mode, on utilise le pigment organique D&C Red n° 28.

Comme exemples de pigments composites utilisables selon l'invention, seuls ou en mélange, on peut citer notamment :
- dioxyde de titane (Cl77891), laque bleue FD&C Blue Aluminium lake (Cl42090) et polymethylhydrogensiloxane (58.1/40.7/1.2)
- dioxyde de titane (Cl77891), D&C Red n° 7 (Cl15850) et polymethylhydrogensiloxane (65.8/32.9/1.3)
- dioxyde de titane (Cl77891), D&C Red n° 28 (Cl45410) et polymethylhydrogensiloxane (65.8/32.9/1.3)
- dioxyde de titane (Cl77891), laque jaune FD&C Yellow 5 Aluminium lake (Cl191140) et polymethylhydrogensiloxane (65.8/32.9/1.3).

Avantageusement, les pigments peuvent être présents sous une forme enrobée hydrophobe dans l'émulsion selon l'invention. Il s'agit plus particulièrement de pigments traités en surface avec un agent hydrophobe pour les rendre compatibles avec la phase grasse de l'émulsion, notamment pour qu'ils aient une bonne mouillabilité avec les huiles de la phase grasse. Ainsi, ces pigments traités sont bien dispersés dans la phase grasse.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme alkyle mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

### Nacres

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

### Matériaux à effet optique

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques, comme, par exemple, les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple, les bronzes et les laitons) sont des métaux préférés.

Par « dérivés métalliques », on désigne des composés dérivés de métaux, notamment, des oxydes, des fluorures, des chlorures et des sulfures

L'agent de coloration goniochromatique peut être choisi, par exemple, parmi les structures multicouches interférentielles et les agents de coloration à cristaux liquides.

### GALENIQUE

Outre les composés décrits précédemment, l'émulsion eau-dans-huile selon l'invention comprend une phase aqueuse dispersée dans une phase grasse liquide, et comprend au moins un milieu physiologiquement acceptable.

Selon un mode particulier de l'invention, la composition de l'invention a un ratio phase aqueuse sur phase grasse liquide supérieur à 1, de préférence supérieur à 1,5, de préférence supérieure à 2.

Par « milieu physiologiquement acceptable », on entend désigner un milieu convenant particulièrement à l'application d'une composition de l'invention sur les matières kératiniques, notamment la peau et les lèvres.
Le milieu physiologiquement acceptable est généralement adapté à la nature du support sur lequel doit être appliquée la composition, ainsi qu'à l'aspect sous lequel la composition doit être conditionnée.

### PHASE AQUEUSE

Une composition selon l'invention comprend au moins une phase aqueuse, de préférence en une teneur allant de 10 à 80 %, de préférence de 30 à 70 %, de façon plus préférentielle entre 40 et 60% en poids par rapport au poids total de la composition.

La phase aqueuse comprend de l'eau et/ou au moins un solvant hydrosoluble.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Le ou les solvant(s) hydrosolubles convenant à l'invention peu(vent)t être choisi(s) parmi les monoalcools en C₁₋₈, et notamment en C₁₋₅, notamment l'éthanol, l'isopropanol, le tert-butanol, le n-butanol, les polyols tels que décrits précédemment, et leurs mélanges. Conviennent aussi tout particulièrement à l'invention, l'éthanol et l'isopropanol et de préférence l'éthanol.

Une composition de l'invention peut en outre comprendre au moins un sel, par exemple, le chlorure de sodium, le chlorure de magnésium et le sulfate de magnésium.

Une composition de l'invention peut comprendre de 0,05 % à 1,5 % en particulier de 0,1 % à 1,0 % et plus particulièrement de 0,15 % à 0,8 % en poids de sels, par rapport au poids total de la composition.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

### PHASE GRASSE LIQUIDE

Une composition cosmétique conforme à la présente invention comprend au moins une phase grasse liquide comprenant au moins un ou plusieurs alcanes linéaires volatiles tels que définis ci-dessus et éventuellement au moins une huile additionnelle.

Une composition de l'invention peut comprendre une phase grasse liquide en une teneur variant de 10 à 80 %, en particulier de 15 à 60 %, et plus particulièrement, de 15 à 40 % en poids par rapport au poids total de la composition.

Selon un mode particulier de l'invention, la composition de l'invention aura un ratio phase aqueuse sur phase grasse liquide supérieur à 1, de préférence supérieur à 1,5, de préférence supérieure à 2.

Une composition selon l'invention peut comprendre au moins une huile additionnelle, choisie parmi les huiles volatiles et non volatiles de type hydrocarbonées, siliconées, ou fluorées. Les huiles peuvent être d'origine animale, végétale, minérale ou synthétique.

On entend par huile, tout corps gras sous forme liquide à température ambiante (20-25 °C) et à pression atmosphérique.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et de préférence allant de 170 °C à 250 °C.

Avantageusement, la phase grasse comprend de 5 % à 40 % en poids, de préférence allant de 7 % à 30 % en poids, et préférentiellement allant de 10 % à 20 % en poids d'huile(s) volatile(s) par rapport au poids total de la composition.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor ; elle peut contenir des groupes ester, éther, amine, amide.

Par huile siliconée, on entend une huile contenant au moins un atome de silicium, et notamment contenant des groupes Si-O.

Par huile fluorée, on entend une huile contenant au moins un atome de fluor.

### Huiles volatiles additionnelles

L'huile volatile hydrocarbonée utilisable dans l'invention peut être choisie parmi les huiles hydrocarbonées ayant un point éclair allant de 40 °C à 102 °C, de préférence allant de 40 °C à 55 °C, et préférentiellement allant de 40 °C à 50 °C.

Comme huile volatile hydrocarbonée, on peut citer les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, l'isohexadécane et par exemple les huiles vendues sous les noms commerciaux d'lsopars ou de Permetyls, les esters ramifiés en C₈-C₁₆ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile siliconée utilisable dans l'invention peut être choisie parmi les huiles siliconées ayant un point éclair allant de 40 °C à 102 °C, de préférence ayant un point éclair supérieur à 55 °C et inférieur ou égal à 95 °C, et préférentiellement allant de 65°C à 95 °C.

Comme huile volatile siliconée, on peut citer les huiles de silicones linéaires ou cycliques ayant de 2 à 7 atomes de silicium, ces silicones comportant éventuellement des groupes alkyle ou alkoxy ayant de 1 à 10 atomes de carbone. Comme exemple d'huile volatile siliconée, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

L'huile volatile additionnelle peut être présente en une teneur allant de 5 % à 30 % en poids, par rapport au poids total de l'émulsion, de préférence allant de 8 % à 20 % en poids, et préférentiellement allant de 10 % à 15 % en poids par rapport au poids total de la composition. La phase grasse de l'émulsion selon l'invention peut comprendre en outre au moins une huile non volatile.

### Huiles non volatiles

Cette huile non volatile ou un de ses mélanges peut être présente en une teneur allant de 1 % à 30 % en poids, par rapport au poids total de l'émulsion, et de préférence allant de 2 % à 20 % en poids.

L'huile non volatile peut être choisie parmi les huiles d'origine minérale, animale, végétale ou synthétique, carbonées, hydrocarbonées et/ou siliconées, et leurs mélanges, dans la mesure où elles sont compatibles avec l'utilisation envisagée.

On peut citer les huiles hydrocarbonées non volatiles telles que l'huile de paraffine ou de vaseline, l'isoeicosane, de soja, le perhydrosqualène, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol.

Comme huile non volatile siliconée, on peut citer les polydiméthylsiloxane (PDMS), éventuellement phénylées telles que les phényltriméthicones, ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, et leurs mélanges.

Selon un mode particulier, on utilisera de l'isononanoate d'isononyle.

### AGENTS TENSIOACTIFS ADDITIONNELS

Une composition selon l'invention comprend un élastomère de silicone émulsionnant et éventuellement un ou plusieurs agent(s) tensioactif(s) additionnels adaptés aux émulsions E/H, notamment choisi(s) parmi les agents tensioactifs amphotères, anioniques, cationiques ou non ioniques, utilisés seuls ou en mélange.

Les agents tensioactifs sont généralement présents dans la composition, en une teneur pouvant aller de 0,5 à 15 % en poids, en particulier de 1,0 à 5% en poids, par rapport au poids total de la composition.

A titre d'exemple, le ou les tensioactif(s) siliconé(s) peut/peuvent être présent(s) en une teneur allant de 0,5 à 10 % en poids, notamment de 1 à 5 % en poids, par rapport au poids total de la composition.

Selon un autre mode de réalisation particulier, le ou les tensioactif(s) non siliconé(s) peut/peuvent être présent(s) en une teneur allant de 0.1 à 10 % en poids, notamment de 0.5 à 8 % en poids, par rapport au poids total de la composition.

Pour les émulsions E/H, on peut utiliser notamment des agents tensioactifs hydrocarbonés ou siliconés.

On peut citer par exemple comme agents tensioactifs hydrocarbonés, les polyesters de polyols comme le PEG-30 dipolyhydroxystearate vendu sous la référence ARLACEL P 135 par la société Uniqema, le polyglyceryl-2 dipolyhydroxystearate vendu sous la référence DEHYMULS PGPH par la société Cognis.

On peut citer par exemple comme agents tensioactifs siliconés les alkyl-diméthicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt.

On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple le polyglyceryl-3 diisostéarate commercialisé sous la dénomination de LAMEFORM TGI par la société Cognis, l'isostéarate de polyglycérol-4, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Selon un mode de réalisation particulièrement préféré, une émulsion selon l'invention, en particulier une émulsion E/H comprenant une phase huileuse siliconée, comprend au moins un tensioactif siliconé, et plus particulièrement choisi parmi les diméthicone copolyols.

La présence d'un diméthicone copolyol favorise notamment la stabilisation de l'émulsion selon l'invention.

Un diméthicone copolyol utilisable selon l'invention est un polydiméthyl méthyl siloxane oxypropyléné et/ou oxyéthyléné.

On peut utiliser comme diméthicone copolyol ceux répondant à la formule (II) suivante : 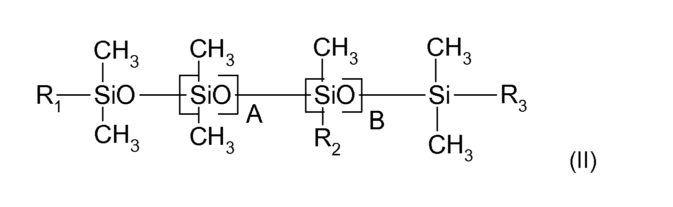 dans laquelle :
- R₁, R₂, R₃, indépendamment les uns des autres, représentent un radical alkyle en C₁-C₆ ou un radical -(CH₂)ₓ - (OCH₂CH₂)y - (OCH₂CH₂CH₂)_{Z} - OR₄, au moins un radical R₁, R₂ ou R₃ n'étant pas un radical alkyle ; R₄ étant un hydrogène, un radical alkyle en C₁-C₃ ou un radical acyle en C₂-C₄;
- A est un nombre entier allant de 0 à 200 ;
- B est un nombre entier allant de 0 à 50 ; à la condition que A et B ne soient pas égaux à zéro en même temps ;
- x est un nombre entier allant de 1 à 6 ;
- y est un nombre entier allant de 1 à 30 ; et
- z est un nombre entier allant de 0 à 5.

Selon un mode de réalisation préféré de l'invention, dans le composé de formule (II), R₁ = R₃ = radical méthyle, x est un nombre entier allant de 2 à 6 et y est un nombre entier allant de 4 à 30. R₄ est en particulier un hydrogène.

On peut citer, à titre d'exemples de composés de formule (II), les composés de formule (III) : 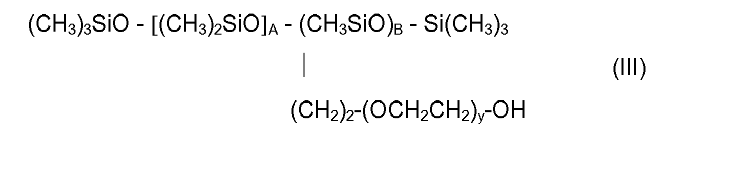 dans laquelle A est un nombre entier allant de 20 à 105, B est un nombre entier allant de 2 à 10 et y est un nombre entier allant de 10 à 20.

On peut également citer à titre d'exemple de composés siliconés de formule (II), les composés de formule (IV) : 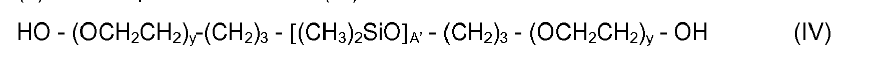 dans laquelle A' et y sont des nombres entiers allant de 10 à 20.

On peut utiliser comme diméthicone copolyols ceux vendus sous les dénominations DC 5329, DC 7439-146, DC 2-5695, Q4-3667 par la société Dow Corning ; KF-6013, KF-6015, KF-6016, KF-6017, KF-6028 par la société Shin-Etsu.

Les composés DC 5329, DC 7439-146, DC 2-5695 sont des composés de formule (III) où respectivement A est 22, B est 2 et y est 12 ; A est 103, B est 10 et y est 12 ; A est 27, B est 3 et y est 12.

Selon un mode de réalisation particulier, le tensioactif siliconé peut être le PEG-polydiméthylsiloxyéthyldiméthicone, notamment commercialisé par la société Shin-Etsu sous la référence KF-6028, le PEG-1 0 diméthicone notamment commercialisé par la société Shin-Etsu sous la référence KF-6017, et leurs mélanges.

Le diméthicone copolyol peut être présent dans la composition selon l'invention en une teneur allant de 0,5 % à 6 % en poids, par rapport au poids total de l'émulsion, et de préférence allant de 1,0 % à 3 % en poids.

Selon un mode particulier de l'invention, la composition comprend, outre l'élastomère de silicone émulsionnant, au moins un tensioactif siliconé et au moins un tensioactif hydrocarboné.

De préférence, ledit tensioactif siliconé est choisi parmi les dimethicone copolyols et ledit tensioactif hydrocarboné est choisi parmi les esters alkylés de polyols.

### Adjuvants cosmétiques

Les compositions de l'invention peuvent contenir en outre un ou plusieurs des adjuvants habituels dans les domaines cosmétique et dermatologique, tels que des agents hydratants ; des émollients ; des actifs hydrophiles ou lipophiles ; des agents anti-radicaux libres ; des séquestrants ; des antioxydants ; des conservateurs ; des agents alcanisants ou acidifiants ; des parfums ; des gélifiants hydrophiles ; des agents filmogènes, en particulier des polymères filmogènes (pour des compositions dans un axe tenue) ; des filtres solaires organiques ou physiques, des colorants hydrosolubles ou liposolubles; et leurs mélanges. Les quantités de ces différents adjuvants sont celles classiquement utilisées dans les fonds de teint.

La composition de l'invention pourra ainsi comprendre en outre au moins un agent filmogène en phase aqueuse et/ou en phase huileuse pour renforcer la tenue de la composition.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

Ces adjuvants sont généralement présents dans la composition en une teneur allant de 0,01 à 20 % en poids, de préférence de 0,1 à 10 % en poids par rapport au poids total de ladite composition.

Selon un mode particulier, la composition de l'invention ne comprend pas d'agent gélifiant et/ou d'agent structurant.

L'invention est illustrée dans les exemples présentés ci-après à titre illustratif et non limitatif du domaine de l'invention :
Sauf indication contraire, les valeurs dans les exemples ci-dessous sont exprimées en % en poids par rapport au poids total de la composition.

### EXEMPLES

### Exemple 1 : Exemple de Fond de Teint selon l'invention et exemple comparatif

| **Phase** | | **Invention** | **Comparatif** |
|---|---|---|---|
| A1 | Elastomère de silicone émulsionnant (1) | 2,50 | 2,50 |
| | Dimethicone 6cst | 6,80 | 12,80 |
| A2 | Mélange majoritaire de n-undécane : n-tridécane dans | 6,00 | 0,00 |
| | lequel le n-undécane est majoritaire dans le mélange* | | |
| | Isododecane | 5,00 | 5,00 |
| | Isononyl isonanoate | 5,00 | 5,00 |
| | Di isostéarate de polyglycerol | 1,00 | 1,00 |
| | PEG-10 Dimethicone (2) | 1,50 | 1,50 |
| | Oxyde de Fer traité en surface (3) | 3,40 | 3,40 |
| | Oxyde de Titane traité en surface (3) | 8,60 | 8,60 |
| | Nano-Oxyde de Titane traité en surface (4) | 4,00 | 4,00 |
| A3 | Poudre de Nylon | 2,00 | 2,00 |
| | Silice Sphérique | 2,00 | 2,00 |
| B | Eau | 44,50 | 44,50 |
| | Sulfate de Magnésium | 0,50 | 0,50 |
| | Conservateur | 1,20 | 1,20 |
| | Propylene Glycol | 2,00 | 2,00 |
| | Butylene Glycol | 4,00 | 4,00 |
| | TOTAL | 100,00 | 100,00 |

| | | | |
|---|---|---|---|
| (1) KSG 210 de Shinetsu * tel que préparé selon la demande WO2008/155059 (2) KF6017 de Shinetsu (3) Pigments NAI de Miyoshi (4) Pigment Mibrid SAS TTO-S3 de Miyoshi | | | |

La phase A1 est obtenu en diluant l'élastomère de silicone dans la diméthicone. Les pigments sont ensuite broyés dans les huiles de la phase A2 et ajouté à la phase A1. Les ingrédients de la phase aqueuse B sont mélangés et on verse ensuite la phase B sur la phase A pour former l'émulsion.

Les deux formules ont un ratio phase aqueuse sur phase grasse liquide de 50,5/22,8.

La formule selon l'invention est très fluide alors que l'exemple comparatif est très épais.

Le Fond de Teint selon l'invention est plus glissant, s'applique plus facilement et se travaille plus longtemps que l'exemple comparatif.

### Exemple 2 : Exemple de Fond de Teint

| **Phase** | | |
|---|---|---|
| A1 | Elastomère de silicone émulsionnant (1) | 2,00 |
| | Elastomère de silicone (2) | 0,75 |
| | Dimethicone 6cst | 7,50 |
| | Mélange majoritaire de n-undécane : n-tridécane | |
| A2 | dans lequel le n-undécane est majoritaire dans le | 5,00 |
| | mélange* | |
| | Isododecane | 5,00 |
| | Isononyl isonanoate | 4,00 |
| | Di isostéarate de polyglycerol | 0,75 |
| | PEG-10 Dimethicone (3) | 1,00 |
| | Oxyde de Fer traité en surface (4) | 3,90 |
| | Oxyde de Titane traité en surface (4) | 10,10 |
| | Nano-Oxyde de Titane traité en surface (5) | 3,00 |
| A3 | Poudre de Nylon | 1,50 |
| | Silice Sphérique | 1,50 |
| | Séricite | 6,00 |
| B | Eau | 42,00 |
| | Sulfate de Magnésium | 0,50 |
| | Conservateur | 1,00 |
| | Propylene Glycol | 1,50 |
| | Butylene Glycol | 3,00 |
| | TOTAL | 100,00 |

| | | |
|---|---|---|
| (1) KSG 210 de Shinetsu (2) KSG 16 de Shinetsu * tel que préparé selon la demande WO2008/155059 (3) KF6017 de Shinetsu (4) Pigments NAI de Miyoshi (5) Pigment Mibrid SAS TTO-S3 de Miyoshi | | |

La phase A1 est obtenu en diluant les élastomère dans la diméthicone. Les pigments sont ensuite broyés dans les huiles de la phase A2 et ajouté à la phase A1. Les ingrédients de la phase aqueuse B sont mélangés et on verse ensuite la phase B sur la phase A pour former l'émulsion.

Le ratio phase aqueuse sur phase grasse liquide de cette formulation est de 46,5/21,5.

Le fond de teint ainsi obtenu est fluide, stable, avec un bon effet matifiant, un toucher très doux et une bonne tenue.

### Exemple 3 : Exemple de Fond de Teint

| **Phase** | | **Formule sur 100g** |
|---|---|---|
| A1 | Elastomère de silicone émulsionnant (1) | 3,00 |
| | Dimethicone 6cst | 6,80 |
| A2 | Mélange majoritaire de n-undécane : n-tridécane dans lequel le n- | 6,00 |
| | undécane est majoritaire dans le mélange* | |
| | Isododecane | 5,00 |
| | Isononyl isonanoate | 5,00 |
| | Di isostéarate de polyglycerol | 1,00 |
| | PEG-10 Dimethicone (2) | 1,50 |
| | Oxyde de Fer traité en surface (3) | 2,30 |
| | Oxyde de Titane traité en surface (3) | 7,70 |
| A3 | Poudre de Nylon | 1,00 |
| | Silice Sphérique | 1,00 |
| | Nacre Mica Titane Blanche | 2,00 |
| B | Eau | 51,00 |
| | Sulfate de Magnésium | 0,50 |
| | Conservateur | 1,20 |
| | Propylene Glycol | 2,00 |
| | Pentylene Glycol | 3,00 |
| | TOTAL | 100,00 |

| | | |
|---|---|---|
| (1) KSG 210 de Shinetsu * tel que préparé selon la demande WO2008/155059 (2) KF6017 de Shinetsu (3) Pigments NAI de Miyoshi | | |

La phase A1 est obtenu en diluant l'élastomère dans la diméthicone. Les pigments sont ensuite broyés dans les huiles de la phase A2 et ajouté à la phase A1. Les ingrédients de la phase aqueuse B sont mélangés et on verse ensuite la phase B sur la phase A pour former l'émulsion.

Le fond de teint ainsi obtenu est fluide, stable, avec un résultat satiné et lumineux, un toucher très doux et une bonne tenue.

Le ratio phase aqueuse sur phase grasse liquide de cette formulation est de 56/22,8.

### Exemple 4 : Exemple de Fond de Teint

| **Phase** | | **Formule sur 100g** |
|---|---|---|
| A1 | Elastomère de silicone émulsionnant (1) | 3,00 |
| | Dimethicone 6cst | 6,80 |
| A2 | Dodécane (Parafol 12 de Sasol) | 6,00 |
| | Isododecane | 5,00 |
| | Isononyl isonanoate | 5,00 |
| | Di isostéarate de polyglycerol | 1,00 |
| | PEG-10 Dimethicone (2) | 1,50 |
| | Oxyde de Fer traité en surface (3) | 2,30 |
| | Oxyde de Titane traité en surface (3) | 7,70 |
| A3 | Poudre de Nylon | 1,00 |
| | Silice Sphérique | 1,00 |
| | Nacre Mica Titane Blanche | 2,00 |
| B | Eau | 51,00 |
| | Sulfate de Magnésium | 0,50 |
| | Conservateur | 1,20 |
| | Propylene Glycol | 2,00 |
| | Pentylene Glycol | 3,00 |
| | TOTAL | 100,00 |

| | | |
|---|---|---|
| (1) KSG 210 de Shinetsu * tel que préparé selon la demande WO2008/155059 (2) KF6017 de Shinetsu (3) Pigments NAI de Miyoshi | | |

La composition est préparée comme indiqué à l'exemple 3.

Le fond de teint est fluide, stable, avec un toucher très doux et une bonne tenue.

Le ratio phase aqueuse sur phase grasse liquide de cette formulation est de 56/22,8.

### Exemple 5 : Exemple de Fond de Teint selon l'invention et exemple comparatif

| **Phase** | | **Invention** | **Comparatif** |
|---|---|---|---|
| A1 | Elastomère de silicone émulsionnant (1) | 4,00 | 0,00 |
| | Cetyl PEG/PPG-10/1 Dimethicone (2) | 0,00 | 1,00 |
| | Dimethicone 6cst | 6,50 | 9,50 |
| A2 | Mélange n-undécane : n-tridécane dans lequel le n- | 6,50 | 6,50 |
| | undécane étant majoritaire dans le mélange* | | |
| | Isododecane | 5,00 | 5,00 |
| | Isodecyl Neopentanoate | 5,00 | 5,00 |
| | Di isostéarate de polyglycerol | 1,00 | 1,00 |
| | PEG-10 Dimethicone (3) | 1,50 | 1,50 |
| | Oxyde de Fer traité en surface (4) | 2,40 | 2,40 |
| | Oxyde de Titane traité en surface (4) | 11,60 | 11,60 |
| A3 | Nitrure de Bore | 2,00 | 2,00 |
| B | Eau | 47,00 | 47,00 |
| | Sulfate de Magnésium | 0,50 | 0,50 |
| | Conservateur | 1,00 | 1,00 |
| | Propylene Glycol | 2,00 | 2,00 |
| | Glycerin | 4,00 | 4,00 |
| | TOTAL | 100,00 | 100,00 |

| | | | |
|---|---|---|---|
| (1) KSG 210 de Shinetsu (2) Abil EM90 de Evonik Goldschmidt * tel que préparé selon la demande WO2008/155059 (3) KF6017 de Shinetsu (4) Pigments NAI de Miyoshi | | | |

La phase A1 est obtenue en diluant l'élastomère de silicone lorsque présent (invention) dans la diméthicone. Les pigments sont ensuite broyés dans les huiles de la phase A2 et ajouté à la phase A1. Les ingrédients de la phase aqueuse B sont mélangés et on verse ensuite la phase B sur la phase A pour former l'émulsion.

Etude de la stabilité: L'aspect macroscopique et microscopique de la composition (qualité de l'émulsion), est évalué 24 heures après la préparation de la composition, ainsi qu'après 2 mois de stockage.

La composition selon l'invention est fluide, très stable et se donne un maquillage unifiant. L'exemple comparatif est très fluide et l'émulsion a une très mauvaise stabilité : un relargage d'huile important apparaît au bout de quelques jours.

Ces résultats confirment que l'utilisation d'un élastomère de silicone émulsionnant selon l'invention (ex : KSG210) dans une émulsion eau-dans-huile en association avec un alcane linéaire volatile notamment en C7-C14, permet d'obtenir une composition fluide et très stable, comparativement à l'utilisation d'un émulsionnant siliconé non élastomérique tel que le Cetyl PEG/PPG-10/1 Dimethicone (ex : Abil EM 90) à taux de matière active équivalente, dans la même architecture.

## Revendications

1. Composition cosmétique de soin et/ou de maquillage des matières kératiniques, en particulier la peau, sous la forme d'une émulsion eau-dans-huile comprenant, dans un milieu physiologiquement acceptable:
(i) au moins un ou plusieurs alcane(s) linéaire(s) volatil(s) notamment en C7-C14, et
(ii) au moins un élastomère de silicone émulsionnant.

2. Composition cosmétique de soin et/ou maquillage des matières kératiniques selon la revendication 1, **caractérisée en ce qu'**elle comprend en outre au moins 10% en poids de matières pulvérulentes, par rapport au poids total de ladite composition.

3. Composition cosmétique de soin et/ou maquillage des matières kératiniques selon la revendication 1 ou 2, **caractérisée en ce que** le ratio phase aqueuse sur phase grasse liquide est supérieur à 1, de préférence supérieur à 1,5, de préférence supérieur à 2.

4. Composition selon l'une quelconque des revendications précédentes, **caractérisée en ce que** ledit alcane linéaire volatil comprend de 7 à 14 atomes de carbone, en particulier de 9 à 14 atomes de carbone, et plus particulièrement de 11 à 14 atomes de carbone.

5. Composition selon l'une quelconque des revendications précédentes, **caractérisée en ce que** l'alcane linéaire volatil est choisi parmi le n-heptane, le n-octane, le n-nonane, le n-undécane, le n-dodécane, le n-tridécane, le n-tétradécane, et leurs mélanges.

6. Composition selon l'une quelconque des revendications précédentes, **caractérisée en ce que** l'alcane linéaire volatil est le dodécane.

7. Composition selon l'une quelconque des revendications précédentes, **caractérisée en ce qu'**elle comprend au moins deux alcanes linéaires volatiles distincts, différant entre eux d'un nombre de carbone n d'au moins 1, en particulier différant entre eux d'un nombre de carbone de 1 ou de 2.

8. Composition selon l'une quelconque des revendications précédentes, **caractérisée en ce qu'**elle comprend un mélange d'au moins deux alcanes linéaires volatiles comprenant
- de 50 à 90% en poids, de préférence de 55 à 80% en poids, préférentiellement encore de 60 à 75% en poids d'alcane linéaire volatil en Cn avec n allant de 7 à 14
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14,
par rapport au poids total des alcanes dans ledit mélange.

9. Composition selon la revendication précédente, **caractérisée en ce qu'**elle comprend un mélange n-undécane : n-tridécane (C11/C13) comprenant
- de 55 à 80% en poids, de préférence de 60 à 75% en poids d'alcane linéaire volatile en C11 (n-undécane)
- de 20 à 45% en poids, de préférence de 24 à 40% en poids d'alcane
linéaire volatile en C13 (n-tridécane)
par rapport au poids total des alcanes dans ledit mélange.

10. Composition selon l'une quelconque des revendications précédentes, **caractérisée en ce qu'**elle comprend de 1 à 30%, de préférence de 2 à 15%, de préférence de 3 à 10% d'alcanes linéaires volatils en poids par rapport au poids total de ladite composition.

11. Composition selon l'une quelconque des revendications précédentes, **caractérisée en ce que** le(s) élastomère(s) de silicone émulsionnants est (sont) choisi(s) parmi les élastomères de silicones polyoxyalkylénés, les élastomères de silicone polyglycérolés, et leurs mélanges.

12. Composition selon l'une quelconque des revendications précédente, **caractérisée en ce qu'**elle comprend en outre un élastomère de silicone non émulsionnant.

13. Composition selon l'une quelconque des revendications précédentes, **caractérisée en ce que** les matières pulvérulentes sont choisies parmi les charges, les pigments, les nacres et leurs mélanges.

14. Composition selon l'une quelconque des revendications précédentes, **caractérisée en ce qu'**elle comprend en outre au moins un agent tensioactif choisi parmi un agent tensioactif siliconé, un agent tensioactif polyglycérolé, et leur mélange.

15. Composition selon l'une quelconque des revendications précédentes, **caractérisée en ce qu'**il s'agit un fond de teint

16. Procédé cosmétique de maquillage et/ou de soin des matières kératiniques, en particulier la peau, comprenant l'application sur lesdites matières kératiniques, d'au moins une composition telle que définie dans l'une des revendications 1 à 15.
